Europäisches Patentamt

(19) European Patent Office

Office européen des brevets

(11) Publication number: **0 107 978**
A1

# (12) EUROPEAN PATENT APPLICATION

(21) Application number: **83306589.9**

(22) Date of filing: **28.10.83**

(51) Int. Cl.³: **C 07 C 43/29**
**C 07 C 41/22**

(30) Priority: **28.10.82 US 437865**

(43) Date of publication of application:
**09.05.84 Bulletin 84/19**

(84) Designated Contracting States:
**AT BE CH DE FR GB IT LI LU NL SE**

(71) Applicant: **ETHYL CORPORATION**
**Ethyl Tower 451 Florida Boulevard**
**Baton Rouge Louisiana 70801(US)**

(72) Inventor: **Ransford, George Henry**
**10 Mallet Hill Court**
**Columbia South Carolina(US)**

(74) Representative: **Baillie, Iain Cameron et al,**
**c/o Ladas & Parry Isartorplatz 5**
**D-8000 München 2(DE)**

(54) Octabromobiphenyl oxide.

(57) The preparation of octabromobiphenyl oxide using molten biphenyl oxide, bromine and a catalyst compound of anhydrous aluminum or iron or compounds thereof. No solvent is necessary. The temperature of the reaction system can be gradually increased to maintain the brominated products in a molten or liquid state. The temperature may range from about 25°C. to about 200°C.

EP 0 107 978 A1

## OCTABROMOBIPHENYL OXIDE PROCESS

This invention relates to a process for preparing octabromobiphenyl oxide. More particularly, this invention relates to the solventless bromination of molten diphenyl oxide using $Br_2$ or BrCl and an aluminum or iron catalyst to obtain octabromobiphenyl oxide.

Octabromobiphenyl oxide is an additive flame retardant for use in polymer compositions. Octabromobiphenyl oxide is frequently used in acrylonitrile-butandiene-styrene (ABS) because of the low melting point of octabromobiphenyl oxide and its high thermal stability. The second primary reason for its use in ABS is its freedom from bloom.

Octabromobiphenyl oxide, as the term is used herein, is a mixture of brominated biphenyl oxides with a range of 6-10 bromine atoms per molecule. The average number of bromine atoms per molecules varies significantly depending on its preparation. The product range and distribution can therefore be altered by modifying the reaction conditions.

German Patent No. 2,950,877 claims a method of preparing ring-brominated aromatic compounds, such as octabromobiphenyl oxide, by reacting biphenyl oxide with bromine in an inert reaction medium in the presence of a halogenation catalyst. From 2 to 90 weight percent of the total stoichiometric amount of bromine is premixed with part or all of the inert reaction medium and part or all of the catalyst. Then the biphenyl oxide is added to the reaction mixture along with the rest of the bromine and any remaining catalyst or inert reaction medium. The octabromobiphenyl oxide product prepared in this manner has approximately 8.0 to 9.0 bromines per molecule with an average of 8.22.

Alternatively, the polybromination of biphenyl oxide may be carried out in excess bromine to minimize costs and facilitate mixing. This process in excess bromine does not, however, permit any control over the degree of bromination and an exhaustively brominated product, decabromodiphenyl oxide, is produced.

Due to the wide product distribution of what is referred to as octabromobiphenyl oxide, it is desirable to use a process whereby the bromination of biphenyl oxide peaks at approximately eight bromines per molecule.

It has now been discovered that by using molten biphenyl oxide, a stoichiometric amount of $Br_2$ or BrCl and a catalyst composed of aluminum or iron or compounds thereof, a favorable octabromodiphenyl oxide product results. Since this invention is carried out without a solvent, a further advantage is that a solvent recovery step is not necessary. Since a stoichiometric amount of bromine is preferred, recovery of excess bromine is not necessary. This invention therefore affords a more cost effective route to octabromobiphenyl oxide.

This invention is a process for preparing octabromobiphenyl oxide comprising adding from about 7.5 to about 9.0 equivalents of $Br_2$ or BrCl to biphenyl oxide and about 0.05 to about 10.0 weight percent of a catalyst composed of aluminum or iron or compounds thereof without a solvent while maintaining the reaction temperature such that the reaction mixture remains in a molten or liquid state.

The brominating agent is selected from the group consisting of $Br_2$ and BrCl. $Br_2$ is preferred since a slight amount of chlorination may take place if BrCl is used. However, there may be times when an occasional chlorine attached to the aromatic ring may not appreciably affect product performance and BrCl would be a suitable brominating agent. Preferably, the amount of $Br_2$ or BrCl is about 8.0 to about 8.3 equivalents per equivalent of biphenyl oxide. More preferably, a stoichiometric amount of $Br_2$ or BrCl is added to the biphenyl oxide, that is 8.0 equivalents of $Br_2$ or BrCl per equivalent of biphenyl oxide. Generally, the bromines are randomly distributed on the aromatic rings of the biphenyl oxide molecule.

Either aluminum or iron or a compound thereof may be used as a catalyst in the practice of the present invention to obtain the desired octabromobiphenyl product. The iron may be in the form of iron powder, $FeCl_3$, $FeBr_3$ and the like. The aluminum may be in the form of aluminum powder, $AlCl_3$, $AlBr_3$, and the like. Aluminum is the preferred catalyst and $AlCl_3$ is more preferred. Only anhydrous catalysts are suggested in the practice of the present invention since catalyst inactivation occurs when water is present.

About 0.05 weight percent to about 10.0 weight percent of a catalyst containing anhydrous aluminum or iron or compounds thereof is used based on the amount of biphenyl oxide charged. Preferably, about 0.5 to about 3.0 weight percent catalyst is used. More preferably, the amount of catalyst is about 0.75 to about 1.25 weight percent.

No solvent is used in the practice of the present invention since the reaction mixture is always maintained in a liquid or molten state. The melting point of biphenyl oxide is about 27°C. Therefore, the reaction mixture is heated to this temperature before any product is obtained. Generally, the biphenyl oxide and catalyst are combined and heated until a molten mass is obtained, usually at least 27°C. Then the bromine is slowly added. The brominated products have higher melting points than 27°C., therefore, as these products are produced, the temperature of the reaction system, beyond the initial exotherm, must be increased accordingly. Alternatively, the reaction mixture may be initially heated to a high enough temperature so that even the brominated biphenyl oxide products which are produced during the reaction remain in the molten or liquid phase. The temperature of the reaction system can range anywhere from about 27°C. to about 200°C. Preferably, the temperature is between about 27°C. and about 150°C. More preferably, the temperature is between about 90°C. and about 120°C.

In a preferred embodiment the octabromobiphenyl oxide reaction product of the present invention contains an average of about 7.6 bromines per molecule of biphenyl oxide. The average molecular weight generally falls within the range of about 750 to about 800. The usual molecular weight of the octabromobiphenyl oxide reaction product prepared by the process of the present invention is about 773.

The following examples represent the process of producing octabromobiphenyl oxide according to the present invention. However, it is to be understood that the practice of this invention is not limited to these examples and other examples are possible.

### EXAMPLE 1

A 500 ml flask was charged with 34.0g biphenyl oxide (0.2 mole BPO) and 0.4g anhydrous $AlCl_3$, under nitrogen, and warmed to 35°C. Then 255.7g $Br_2$ (1.6 mole) was added at a rate of about 1 ml/min. HBr evolution slowed significantly after about half the $Br_2$ had been added. $Br_2$ addition was stopped after 45 minutes and the mixture was allowed to remain at 60°C. for one hour. A significant amount of $Br_2$ was collected overhead. Since catalyst deactivation by wet $Br_2$ was suspected, an additional 1.2 g of anhydrous $AlCl_3$ was added. The remainder of the $Br_2$ was added over 1 1/4 hours at 90-100°C. After 0.5 hours at 100°C., the total HBr evolved was 114.2g (88% of theory for 8 HBr). An additional 32g $Br_2$ (0.2 mole) was added over one hour at 105-110°C. The mixture was allowed to remain at 115°C. for 0.5 hour. The total HBr overhead was 128.6g (99% for 8 HBr).

The viscous, black mixture was heated to 120°C. and added slowly, with vigorous stirring, to 400 ml water. The resulting granular product was filtered, washed, dried and pulverized to give 148.6g of a cream-colored powder, melting point 87-115°C. Yield was 92.8% based on $Br_8$. The gas chromatograph area percent composition is given in Table 1.

EXAMPLE 2

The equipment and set-up was identical to that for Example 1 except a 500 ml resin flask was used. The flask was charged with 68.0g biphenyl oxide (0.4 mole BPO) and 0.7g (1 wt.%) of anhydrous $AlCl_3$ under a slow $N_2$ purge. The mixture was warmed to 35°C., and then 511.4g $Br_2$ (3.2 mole) was added at such a rate as to minimize bromine loses overhead. Initially, this rate was about 1.2 ml/min, but slowed to about 0.5 ml/min as the reaction progressed. The temperature increased to 60°C. due to the reaction exotherm, and was further increased to 115°C. by external heating. After the $Br_2$ addition was complete (about 4.5 hrs. total), an additional 25.4g $Br_2$ (0.16) was added to the 115°C. reaction mixture at a rate of about 0.2 ml/min. After addition was again complete, the mixture was allowed to remain at 150°C. for 0.5 hr. Total HBr evolved: 242.3g (93% theory for 8 HBr). $Br_2$ recovered from cold traps: 37.8g, for a net amount reacted of 499g (7.8 equivalents). The resulting dark melt was added slowly, with vigorous stirring to 1.3 liters of water. The resulting granular product was filtered, washed with water, and dried under vacuum (2.0 mm) for 5 hrs. at 45°C. to give 292.2g (91.9% yield based on $Br_8$) of tan product. This material was ball-milled overnight. The resulting tan powder had a slight odor of bromine and a melting range of 78-88°C. with effervescence at 140°C. The gas chromatograph area % composition is given in Table 1.

EXAMPLE 3

The equipment and set up was similar to that used in Example 2. The flask was charged with 68.0g biphenyl oxide (0.4 mole BPO) and 1.7g anhydrous $AlCl_3$ (2.5 wt.% of biphenyl oxide charge) under $N_2$. The funnel was charged with 543.3g $Br_2$ (3.4 mole) and the $Br_2$ was added initially

at a rate of about 0.6 ml/min. After about 3/4 of the $Br_2$ had been added, the rate was slowed to about 0.2 ml/min. The reaction temperature was gradually increased from 25°C. to 110°C. during the addition and the mixture was allowed to remain at 110°C. under a slow $N_2$ purge overnight. The viscous black mixture was then allowed to remain at 130°C. for an additional 6 hrs. Eventually the reaction mixture became gray. Total HBr liberated: 257.8g (99% of theory for 8 HBr). A total of 31.4g of $Br_2$ was also recovered from the cold traps for a net $Br_2$ reacted value of 8.0 equivalents. The hot gray melt was added slowly to vigorously stirred 10% NaOH (2 liters). The resulting granular product was filtered, washed with $H_2O$ until the washings were neutral, and air dried overnight to yield 290.1g (90.4%) of an off-white powder following grinding, melting point 89-98°, effervescence at 140°C. The gas chromatograph area % composition is given in Table 1.

TABLE 1

| Example | 1 | 2 | 3 |
|---|---|---|---|
| $Br_2$ moles/mole BPO | 8.4 | 7.8 | 8.0 |
| $AlCl_3$, g per g BPO | 0.04 | 0.01 | 0.025 |
| Melting Range, °C | 122-42 | 78-88 | 89-98 |
| Yield % based on BPO charge and $Br_8$ product | 92.8 | 91.9 | 90.4 |
| GC Area % | | | |
| $Br_6$ | ~2 | 7.3 | 4.5 |
| $Br_7$ | 30.7 | 40.0 | 34.4 |
| $Br_8$ | 53.4 | 36.5 | 48.3 |
| $Br_9$ | 14.3 | 14.7 | 12.2 |
| $Br_{10}$ | <1 | 1.3 | 0.7 |
| Avg. Br/molecule | 7.84 | 7.61 | 7.71 |
| $H_2O$, wt % | 0.8 | 0.3 | 0.4 |
| Br, wt % ring | 78.0 | 79.4 | 81.1 |
| Al, ppm | 525 | 265 | - |
| Color, APHA | 110 | 148 | - |
| Viscosity, centistokes | | | |

0107978

-7-

TABLE 1 cont.

| Example | 1 | 2 | 3 |
|---|---|---|---|
| 125°C. | solid | almost solid | - |
| 150°C. | 230.3 | 53.7 | - |
| 170°C. | 42.3 | 18.7 | - |
| Heat capacity, cal/g/°C (at 125°C) | 0.0310 | - | - |

## EXAMPLE 4

This experiment used a similar set-up to those described above. A 500 cc flask was charged, under nitrogen, with 51g biphenyl oxide (0.3 mole) and a 0.51g Fe powder and warmed to 40°C. The funnel was charged with 360g $Br_2$ (2.25 mole) and the $Br_2$ was slowly added to the flask at a rate of about 1.5 ml/min. The mixture exothermed to 90°C. After 45 min, when about half the $Br_2$ had been added, the addition rate was slowed to 0.5 ml/min to minimize foaming and $Br_2$ breakthrough. After $Br_2$ addition was complete (about 2 1/2 hrs.), an additional 23.9g (0.15 mole) $Br_2$ was added (to make up for overhead losses) at 110°C. over 45 min and the resulting viscous dark brown mixture was stirred at 110-125°C. overnight under a slow $N_2$ purge. Total HBr evolved: 172g (94.5% of theory for 8 HBr). The hot, viscous melt was poured slowly, with vigorous stirring, into 400 ml of 2N HCl. The resulting granular product was filtered, washed and dried to give 197.6g (82% based on $Br_8$) of crude product as dark brown pellets, mp 74-80°. A portion (19.7g) was pulverized and vac. dried to give 18.8g of tan powder, mp 75-81°, effervescense at 140°C. GC area % assay indicates a composition of 38.5% $Br_6$, 47.1% $Br_7$ and 12.1% $Br_9$. Total wt % Br = 78.1% (99% of theory for $Br_{7.5}$).

## CLAIMS

1.      A process for preparing octabromobiphenyl oxide characterized by comprising adding from about 7.5 to about 9.0 equivalents of brominating agent consisting of $Br_2$ or BrCl to biphenyl oxide and about 0.05 to about 10.0 weight percent of a catalyst composed of anhydrous aluminum or iron or compounds thereof without a solvent while maintaining the reaction temperature such that the reaction mixture remains in a molten or liquid phase.

2.      A process according to claim 1, characterized in that the catalyst is aluminum.

3.      A process according to claim 2, characterized in that said catalyst is $AlCl_3$.

4.      A process according to claim 3, characterized in that about 0.5 to about 3.0 weight percent $AlCl_3$ catalyst is added.

5.      A process according to any of the preceding claims, characterized in that said temperature is within the range of about 27°C. to about 200°C.

6.      A process according to any of the preceding claims, characterized in that said brominating agent is $Br_2$.

7.      A process according to claim 6, characterized in that about 8.0 to about 8.3 equivalents of said $Br_2$ is added to said biphenyl oxide.

CASE 5131

0107978

## European Patent Office

## EUROPEAN SEARCH REPORT

Application number

EP 83306589.9

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. ³) |
| X | DE - B - 1 253 719 (CHEMISCHE FABRIK KALK G.M.B.H.) <br><br> * Claim 1; column 4, lines 22-25 * <br> -- | 1-5 | C 07 C 43/29 <br> C 07 C 41/22 |
| X | GB - A - 1 436 657 (I.S.C. CHEMICALS LIMITED) <br><br> * Claims 1,2,4-6,9,10 * <br> -- | 1,3,5, 6 | |
| D,A | DE - A1 - 2 950 877 (CHEMISCHE FABRIK KALK GMBH) <br><br> * Claims 1,3; example 5 * <br> -- | 1,5-7 | |
| P,X | GB - A - 2 097 383 (GREAT LAKES CHEMICAL CORPORATION) <br><br> * Claims 1,2,4 * <br> ---- | 1-6 | TECHNICAL FIELDS SEARCHED (Int. Cl. ³) <br><br> C 07 C 41/00 <br> C 07 C 43/00 |

The present search report has been drawn up for all claims

| Place of search | Date of completion of the search | Examiner |
|---|---|---|
| VIENNA | 30-12-1983 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82